# EUROPÄISCHE PATENTANMELDUNG

(11) **EP 0 599 262 A2**
(43) Veröffentlichungstag der Anmeldung: **01.06.1994**
(21) Anmeldenummer: 93118801.5
(22) Anmeldetag: 23.11.1993
(51) Int. Cl.: A61K 37/50

(54) **Stabilisierte Superoxid-Dismutase (SOD)-Zusammensetzung**

(30) Priorität: 27.11.1992 DE 4239877
(71) Anmelder: BOEHRINGER INGELHEIM INTERNATIONAL GmbH, D-55216 Ingelheim am Rhein (DE)
(72) Erfinder: Meyer, Reiner, Dr., D-2344 Maria Enzersdorf (DE)

(57) **Zusammenfassung**

Eine stabilisierte Superoxid-Dismutase (SOD)-Zusammensetzung enthält als Zusätze Cyclodextrin, vorzugsweise Hydroxypropyl-β-cyclodextrin, und entweder einen Zucker oder einen Zuckeralkohol, vorzugsweise Mannitol.

Gefriergetrocknete Formulierungen mit der erfindungsgemäßen Zusammensetzung ergeben bei Resolvatation klare Lösungen mit hoher enzymatischer Aktivität.

## Beschreibung

Die Erfindung betrifft stabilisierte Superoxid-Dismutase (SOD)-Zusammensetzungen, in denen die Bestandteile SOD, ein Cyclodextrin und zusätzlich ein Mono- oder Disaccharid oder ein von einem Monosaccharid abgeleiteter Zuckeralkohol in Kombination vorliegen, pharmazeutische Zusammensetzungen, die diese Komponenten enthalten, die kombinierte Verwendung der Einzelkomponenten zur Herstellung von Arzneimitteln sowie Verfahren zur Herstellung derartiger stabilisierter Zusammensetzungen.

Als Folge verschiedener biochemischer Prozesse in biologischen Systemen (z.B. Redoxprozesse in der Atmungskette, Oxydationen im Cytoplasma) werden bekannterweise laufend O₂.-Radikale gebildet, die hochcytotoxisch sind und zu Gewebezerstörungen führen können. Bei pathologischen Situationen, z.B. im Verlauf rheumatisch bedingter Erkrankungen, werden Degradationen von Collagen und Synovialflüssigkeit durch solche Radikale diskutiert (Pasquier, C. *et al.*, *Inflammation 8*, 27-32, 1984).

Eukaryotische Zellen enthalten hauptsächlich zwei Formen von Superoxiddismutasen (SOD), von denen die eine vornehmlich im Cytosol (Cu/Zn-SOD) und die andere vornehmlich in den Mitochondrien (Mn-SOD) vorliegt. In Lebermitochondrien wurde gefunden, daß das Mn-Enzym in der Matrix einschließlich der inneren Membran lokalisiert ist, obwohl die Mn-SOD auch im Cytosol der Leberzellen nachgewiesen wurde (Mc Cord J.M. *et al.*, in: *Superoxide und Superoxide Dismutases* [A.M. Michelson, J.M. Mc Cord, I. Fridovich, *Hrsg.*.] Academic Press, N.Y., 129-138, 1977).

In Prokaryoten existiert neben einer Mn-SOD noch eine Fe-SOD. Letztere wurde auch in Algen und Protozoen sowie in einigen Pflanzenspezies nachgewiesen (Bridges, S.M., Salin, M.L., *Plant Physiol. 68*, 275-278, 1981).

Diese hochaktiven Enzyme katalysieren die Disproportionierung O₂.⁻ + O₂.⁻ + 2H⁺ → H₂O₂ + O₂ und verhindern durch diese Dismutation der Superoxidradikale deren Anreicherung und somit deren zellschädigende Wirkung. Neben dem endoplasmatischen Retikulum der Leber können die mitochondrialen Membranen als einer der wichtigsten Orte der O₂-Entstehung in tierischen Zellen angesehen werden, sodaß es nicht verwundert, daß Mitochondrien ihre eigene, spezielle SOD (Mn-SOD) zur Verfügung haben.

Das Strukturgen einer prokaryotischen Mn-SOD (*E. coli*) wurde kloniert und das chromosomale *sod*A-Gen lokalisiert (Touati, D., *J Bact. 155*, 1078-1087, 1983).

Die 699 bp lange Nukleotidsequenz einer mitochondrialen Hefe Mn-SOD konnte aufgeklärt und die Primärstruktur sowohl des Precursors als auch des maturen Proteins davon abgeleitet werden - mit Molekulargewichten von 26123 Da für den Precursor und 23059 Da für das mature Protein (Marres, C.A.M. *et al.*, *Eur. J Biochem. 147*, 153-161, 1985). Somit unterscheiden sich die Mn- und Cu/Zn-SOD (MG=14893, EP-A 138111) in ihren Molekulargewichten deutlich.

Die vollständige Aminosäuresequenz der Mn-SOD aus Menschenleber wurde von Barra, D. publiziert, wonach die hMn-SOD aus 196 Aminosäuren zusammengesetzt sein soll (Barra, D. *et al.*, *J. Biol. Chem. 259*, 12595-12601, 1984). Die humane Cu/Zn-SOD aus Erythrocyten besteht demgegenüber aus 153 Aminosäuren (Jabusch, J.R., *et al. Biochemistry 19*, 2310-2316, 1980) und zeigt keine Sequenzhomologien zur hMn-SOD (Barra, D. *et al.*, *loc. cit.*).

Die Herstellung der SOD, insbesondere über die Methoden der DNA-Rekombination, ist aus dem Stand der Technik bekannt und wird vielfältig beschrieben. Beispielsweise kann die humane Cu/Zn-SOD gemäß der EP-A 0 138 111, EP-A 0 180 964, WO 85/01503 oder WO 91/06634 hergestellt werden. Nach dem in der EP-A 0282 899 beschriebenen Verfahren ist die humane Mn-SOD erhältlich. In der EP-A 0 236 385 oder in der EP-A 0 492 447 wird die Herstellung der EC-SOD offenbart. Die Herstellung einer Fe-SOD wird gemäß der EP-A 218 480 ermöglicht und pflanzliche SODs werden in der EP-A 0 359 617, der EP-A 0 356 061 oder in der WO 90/01260 beschrieben. Eine Mn-SOD aus *Serratia* wird in der EP-A 0 210 761 offengelegt.

Die Herstellung und Gewinnung von SOD nach konventionellen Verfahren, beispielsweise durch Extraktion aus Zellen und/oder Geweben tierischer oder menschlicher Herkunft und anschließender Reinigung, beispielsweise über chromatographische Trennung, ist ebenfalls vielseitig beschrieben (z.B. EP-A 0 188 053, DE-OS 3124 228). Darüber hinaus sind chemisch modifizierte Derivate der SOD oder SOD-Analoga bekannt und z.B. in der EP-A 0 292 321 bzw. EP-A 0 483 113 oder WO 89/012677 offenbart.

Demzufolge umfassen die erfindungsgemäß zu verwendenden SODs auch alle Derivate und Analoga der SODs mit dem bekannten biologischen bzw. enzymatischen Aktivitätspektrum.

Bei den erfindungsgemäß zu verwendenden Cyclodextrinen handelt es sich um zyklische Oligosaccharide oder zyklische Polymere, deren Ringsysteme aus sechs, sieben oder acht α-1,4-verknüpften Glucose-Einheiten bestehen, und entsprechend ihrer Anzahl an Monomeren als α-, β- bzw. γ-Cyclodextrine bezeichnet werden. Von den Cyclodextrinen ist bekannt, daß sie mit verschiedenen Biomolekülen, wie z.B. Fettsäuren oder Aminosäuren, Inklusionskomplexe bilden, bzw. diese bis zur Sättigung einschließen können. Aus der Gruppe der Cyclodextrine sind vorzugsweise die β-Cyclodextrine bzw. chemisch modifizierte Derivate davon verwendet worden, um stabile, wässrige Proteinlösungen herzustellen bzw. um deren Toxizität zu überprüfen. Von der sehr großen Anzahl der bisher publizierten Literatur, die sich mit der Herstellung und Verwendung von Cyclodextrinen, insbesondere der ß-Cyclodextrine, befaßt, sollen nur beispielhaft die folgenden Publikationen genannt werden: Manning, M. *et al.*, *Pharm. Res. 6*, 1903-1918, 1989; Yoshida, A. *et al.*, *Int. J. Pharm. 461*, 217-222, 1988; Brewster, M. *et al.*, *Int. J. Pharm. 59*, 231-243, 1980; Pitha, J. *et al.*, *Int. J. Pharm. 29*, 73-82, 1986; Pitha, J. & Pitha J., *J Pharm. Sci. 74*, 987-990, 1985; Brewster, M. *et al.*, *J Parent. Sci. Technol. 43*, 231-240, 1978. Insbesondere wird von Pitha J. & Pitha, J., *loc. cit.* (1986) die Herstellung von Hydroxypropyl-ß-cyclodextrin (HPBCD) beschrieben, und es wird berichtet, daß durch Verwendung von HPBCD die Wasserlöslichkeit der verschiedensten Wirkstoffe und biologischen Marklomoleküle verbessert wird. Hinsichtlich der Verwendung der Cyclodextrine auf pharmazeutischem Gebiet sei auf die Übersichtsartikel von Pitha, J. *et al.*, in: *Controlled Drug Delivery* [Bruck, S.D., *Hrsg.*] Vol. I, CRC Press, Boca Raton, Florida, 125-148, 1983, verwiesen, oder auf Uekama, K., *et al.*, in: CRC *Critical Reviews in Therapeutic Drug Carrier Systems, Vol. 3 (1)*, 1-40, 1987; bzw. auf Uekama, K., in: *Topics in Pharmaceutical Sciences* 1987 [Breimer, D.D. and Speiser, P., *Hrsg.*] Elsevier Science Publishers B.V. (Biomedical Division), 181-194, 1987). Die Verwendung von insbesondere 2-Hydroxypropyl-β-cyclodextrin zur Solubilisierung und Stabilisierung von verschiedenen biologisch aktiven Proteinen wird von Brewster, M.E. *et al.*, *Pharm. Res. 8*, 792-795, 1991, näher beschrieben.

Unter den erfindungsgemäß zu verwendenden Zuckern sind die Monosaccharide, vorzugsweise die Aldohexosen, Ketohexosen und ihre Derivate und optischen Isomere, und die Disaccharide zu verstehen, beispielsweise D(+)-Glucose, D(+)-Mannose, D(+)-Galactose, D(-)-Fructose, D(+)-Sorbose, Saccharose, Lactose, Maltose.

Als Zuckeralkohole kommen die zu den entsprechenden mehrwertigen Alkoholen reduzierten Monosaccharide in Frage, beispielsweise solche, die aus den oben erwähnten Monosacchariden abgeleitet werden können, wobei Mannitol bzw. D-Mannitol (D-Mannit) ganz besonders bevorzugt ist.

Viele Versuche wurden bisher unternommen, biologisch aktive Proteine durch Zusatz verschiedener Substanzen und Gemische zu stabilisieren.

Beispielsweise wird gemäß EP-A 0 142 345 vorgeschlagen, durch Zusatz eines Proteaseinhibitors Interferone zu stabilisieren, oder es wurden Zuckeralkohole für das Erreichen eines Stabilisierungseffektes beschrieben (EP-A 0 080 879). Neben diesen Stabilisatoren wurden auch Humanserumalbumin (EP-A 0 162 332), Dextrane, Dextrine und andere Saccharide (EP-A 0 123 291), Albumin mit Zucker oder Zuckeralkoholen (EP-A 0231 132), Gelatine (EP-A 0 091 258) oder bestimmte Puffersysteme (WO 88/09674) als stabilisierende Mittel bei biologisch aktiven Proteinen (Interferone, TNF, IL-2) offenbart.

Die WO 90/03784 beschreibt die Verwendung von β- und γ-Cyclodextrin-Derivaten, insbesondere von Hydroxypropyl-β-cyclodextrin (HPBCD), zur Stabilisierung verschiedener biologisch aktiver Proteine. In dieser Patentanmeldung wird eine Stabilisierung oder Solubilisierung bzw. Aggregationsverhinderung mit insbesondere HPBCD explizit für Interleukin-2 (IL-2), Tumor-Nekrose-Faktor (TNF), Makrophagen-Kolonie-stimulierenden Faktor (m-CSF), Insulin und humanes Wachstumshormon (HGH) beschrieben.

Der in der WO 90/03784 beschriebene Sachverhalt ist Gegenstand der Publikation von Brewster, M.E. *et al.*, *Pharm. Res. 8*, 792-795, 1991.

Von Lee, M. und Fennema O.R., *J Agric. Food Chem. 39*, 17-21, 1991 ist bekannt, daß Cyclodextrine die Aggregationsbildung von β-Casein verhindern.

Die Publikation von Hora M. S. *et al.*, *Pharm. Res. 9*, 33-36, 1992, offenbart die Verhinderung der Bildung von löslichen Dimeren bei in lyophilisierter Form vorliegendem Tumor-Nekrose-Faktor (TNF), wenn vor der Lyophilisierung dem TNF Mannitol zusammen mit einer amorphen Komponente (Dextran, Saccharose, Trehalose oder 2-Hydroxypropyl-β -cyclodextrin) hinzugegeben wird.

Wegen der potentiellen klinischen Bedeutung von SODs besteht ein Bedarf an stabilisierten Formulierungen dieser Proteine. Dies betrifft insbesondere lyophilisierte SOD-Präparate, die bei der Resolvatisierung klare Lösungen ergeben. Versuche, dies durch Zusätze wie Mannitol, Saccharose, Tween 20®, Tween 80® oder Serumalbumin zu erreichen, schlugen fehl.

Ein besonderes Problem der Superoxid-Dismutasen besteht darin, daß die enzymatisch aktiven Formen Oligomere aus nicht-kovalent assoziierten Untereinheiten sind; im Fall der Cu/Zn-SOD ein Dimer (Hartz & Deutsch, *J Biol. Chem. 247,* 7043-50 (1972)), im Fall der Mn-SOD ein Tetramer (McCord J.M. *et al.*, *Superoxide and Superoxide Dismutases*, Academic Press, London (1977), S. 129-38). Stabilisierende Zusätze sollen zwar die Bildung unlöslicher hochmolekularer Aggregate, nicht jedoch die Aggregation zum enzymatisch aktiven Oligomer verhindern.

Demgegenüber stand kein Verfahren zur Verfügung, ein aus mehreren nicht-kovalent aggregierten Untereinheiten bestehendes Enzym wie Superoxid-Dismutase so zu formulieren, daß die Bildung unlöslicher Aggregate vermieden wird, ohne die Assoziation der Untereinheiten und damit die enzymatische Aktivität zu beeinträchtigen.

Ein besonderes Problem stellte die Behandlung hochkonzentrierter Lösungen (10-60 mg/mi SOD) dar.

Überraschenderweise wurde gefunden, daß der kombinierte Zusatz von HPBCD und einem oder mehreren Mono- oder Disacchariden bzw. von Monosacchariden abgeleiteten Zuckeralkoholen vor der Lyophilisation von SOD-Lösungen die Bildung unlöslicher hochmolekularer Aggregate verhindern konnte, nicht jedoch die erwünschte Aggregation zum enzymatisch aktiven Oligomer. Als besonders geeignet erwies sich die Kombination von HPBCD mit Mannitol.

Die erfindungsgemäßen Formulierungen von Superoxid-Dismutase werden zweckmäßig als gefriergetrocknete Präparate hergestellt. Solche Lyophilisate liefern nach Resolvatisierung klare Lösungen mit hoher enzymatischer Aktivität.

Die zur Gefriertrocknung bestimmten Lösungen können Superoxid-Dismutase in Konzentrationen von 0.1-60 mg/mi enthalten. Bevorzugt sind Konzentrationen von ≧ 5 mg/ml, besonders bevorzugt von ≧ 10 mg/ml. Vorzugsweise enthalten diese Lösungen Hydroxypropyl-β-cyclodextrin in einem Anteil von 0.5-20 % (Gewicht/Volumen) ganz bevorzugt 1-11%, insbesondere 1-6 %, und Mannitol in einem Anteil von 0.5-10 %, ganz bevorzugt 1-5 % (Gewicht/Volumen).

Gegenstand der Erfindung sind ferner Verfahren zur Herstellung der erfindungsgemäßen Formulierungen.

Die erfindungsgemäßen Formulierungen können zur Behandlung von Krankheiten verwendet werden, zum Beispiel Entzündungen, Lungenfibrose, bronchiale pulmonäre Dysplasie, Hyperoxie der Lunge, akute Atemwegserkrankungen (Z. B. *adult respiratory distress syndrome* (ARDS), *infantile respiratory distress syndrome* (IRDS)), Fibrose nach Strahlenexposition, durch Reperfusion nach Ischämie entstandene Schäden, oder zur Verlängerung der Überiebenszeit isolierter Organe *ex vivo.* Entsprechend sind pharmazeutische Zusammensetzungen, die die erfindungsgemäßen Zusammensetzungen sowie pharmazeutisch geeignete Träger- und/oder Hilfsstoffe enthalten, ebenfalls Gegenstand der Erfindung. Gegenstand der Erfindung ist ferner die kombinierte Verwendung der genannten Einzelkomponenten zur Herstellung von Arzneimitteln.

Das folgende Beispiel erläutert die Erfindung.

### Beispiel

### Experimentelles Verfahren zur Herstellung von Lyophilisaten und Resolvaten

Mn-SOD wurde gemäß EP-A 282 899 hergestellt. Hydroxypropyl-β-cyclodextrin ist bei ICN Biomedicals GmbH, Meckenheim, FRG, und D-Mannitol bei Serva Feinbiochemica GmbH & Co., Heidelberg, FRG, erhältlich.

Das SOD-haltige Konzentrat (20 - 40 mg/ml Mn-SOD, 150 mM Natriumchlorid, 10 mM Natriumphosphat, pH 7.2) wurde gegen den Puffer dialysiert, der in der entsprechenden Formulierung verwendet werden sollte (siehe unten). Nach jeweils 2 Stunden wurde die Pufferlösung 3-malig gewechselt. Die Ausschlußgrenze des Dialyseschlauches war für Molekulargewichte ≧ 10000 Da.

Die Dialyse war aber nicht notwendig, wenn die entsprechende Formulierung 10 mM Natriumphosphat und Natriumchlorid im Bereich von 75 - 150 mM enthalten sollte, da die Mn-SOD-Konzentration im Konzentrat hoch genug war, um nach Vermischen mit der Zusatzstoff-Lösung die gewünschte Endkonzentration der Zusatzstoffe erhalten zu können.

Eine Lösung mit Zusätzen wurde in einer Pufferlösung so angesetzt, daß nach dem Vermischen der Mn-SOD-haltigen Lösung mit der die Zusätze enthaltenden Pufferlösung die gewünschten Endkonzentrationen aller Substanzen eingestellt wurden. Die so erhaltene Formulierung wurde zu 1.0 ml in Ampullen abgefüllt. Diese abgefüllten Ampullen wurden lyophilisiert (Einfrieren auf -40°C, 2 Stunden halten bei -40°C, Haupttrocknung bei -30°C und 0.02 mbar bis Produkttemperatur gleich der Stellflächentemperatur, Nachtrocknung bei 0°C 4 Stunden und bei +20°C 2 Stunden; Gefriertrocknungsgerät Edwards/Kniese, Lyofex 0.4).

Nach der Lyophilisation wurden die Ampullen mit Stopfen verschlossen und im Kühlschrank, bei Raumtemperatur und bei 40°C im Dunkeln gelagert. Der gesamte Herstellungsvorgang erfolgte unter sterilen Bedingungen. Nach bestimmten Zeitintervallen (maximal 1/2 Jahr) wurden die Lyophilisate mit Wasser wieder aufgelöst und analysiert. Routinemäßig analysiert wurden die Beschaffenheit der Lyokuchen, das Resolvatverhalten, das Resolvat, die spezifische Mn-SOD-Aktivität (McCord *et* Fridovich, *J. Biol. Chem. 244*, 6049 (1969)) sowie etwaige Abbauprodukte und Aggregate mittels Polyacrylamidgelelektrophorese (Laemmli, *Nature 227*, 680-5 (1970)).

In der beigefügten Tabelle sind eine Reihe von Formulierungen zusammengefaßt, die nach diesem Muster hergestellt wurden. Die getesteten Zusatzstoffe waren: Natriumphosphatpuffer (Na-Phosphat), Natriumphosphat-Natriumcitrat-puffer (Phos-Citrat), Mannitol, Natriumchlorid (NaCl), Tween 80®, Cystein, Saccharose (Sucrose) und Hydroxypropyl-βcyclodextrin (HPBCD). Nach 1-2 Wochen Lagerzeit konnte bei den Formulierungen, die nicht die Kombination aus HPBCD und Mannitol enthielten, schon die ersten Trübungen bei 21° und 40°C Lagerung festgestellt werden. Die Formulierungen, die die Kombination aus HPBCD und Mannitol enthielten, zeigten auch noch bei 40°C nach 7 Monaten Lagerung keine trüben Resolvate.

Neben den in der Tabelle enthaltenen Formulierungen, die mit einer Mn-SOD-Konzentration von 10 mg/ml angesetzt wurden, wurden Formulierungen aus Lösungen hergestellt, die 0.1, 1.0 und 20 mg/ml Mn-SOD enthielten.

In den letzten 3 Spalten sind die Auswertungen der Beobachtungen aufgeführt, die den deutlichsten Unterschied zwischen den Formulierungen zeigten: Trübung der Resolvate nach Lagerung der Lyophilisate bei verschiedenen Temperaturen (5°C, 21°C, 40°C).

**Tabelle**

| *Resolvateigenschaften verschiedener MnSOD-Präparate (Lyophylisate)* | | | | | | | |
|---|---|---|---|---|---|---|---|
| [MnSOD] (mg/ml) | [Mannitol]¹ (%) | [NaCl] (mM) | [HPBCD] (%) | Puffer | Trübung² | | |
| | | | | | 5°C | 21°C | 40°C |
| 10 | | | 5 | Phos-Citrat 10 nM pH 7.2 | 0 | 0 | ++ |
| 10 | 2 | | 6 | Phos-Citrat 10 mM pH 7.2 | 0 | 0 | 0 |
| 10 | 1 | | 11 | Phos-Citrat 10 mM pH 7.2 | 0 | 0 | 0 |
| 10 | | 61 | 6 | Phos-Citrat 10 mM pH 7.2 | 0 | + | ++ |
| 10 | | 34 | 11 | Phos-Citrat 10mM pH 7.2 | 0 | 0 | ++ |
| 10 | 2 | | 6 | Na-Phosphat 10 mM pH 7.2 | 0 | 0 | 0 |
| 10 | | 61 | 6 | Na-Phosphat 10 mM pH 7.2 | 0 | ++ | +++ |
| 10 | 4.5 | | 1 | Na-Phosphat 10 mM pH 7.2 | 0 | 0 | 0 |
| 10 | | 150 | | Na-Phosphat 10 mM pH 7.2 | +++ | | |
| 10 | 5 | | | Na-Phosphat 10 mM pH 7.2 | +++ | +++ | +++ |
| 10 | Sucrose (0.3 %) | 8.6 | | Na-Phosphat 0.5 mM pH 7.2 | + | | |

| | | | | | | | |
|---|---|---|---|---|---|---|---|
| ¹ oder das angegebene Additiv | | | | | | | |
| ² **Trübung**: Die bei 3 Temperaturen (5°c, 21°C, 40°C) gelagerten Trockenampullen wurden nach der Resolvatisation mit dem Auge bemessen, wobei in 4 Stufen das Ausmaß der Trübung eingeteilt werden konnte: 0: klare Lösung; +: wenig Partikel und/oder sehr gering trüb; ++: viele Partikel und/oder gut sichtbar trüb; +++: stark trüb oder flockig | | | | | | | |

## Patentansprüche

1. Lösliche Zusammensetzung enthaltend Superoxid-Dismutase, Cyclodextrin und eine Substanz, die ausgewählt ist aus der Gruppe der Zucker und der Zuckeralkohole.

2. Zusammensetzung nach Anspruch 1, dadurch gekennzeichnet, daß die Superoxid-Dismutase eine Mangan-Superoxid-Dismutase ist.

3. Zusammensetzung nach Anspruch 1, dadurch gekennzeichnet, daß das Cyclodextrin Hydroxypropyl-β-Cyclodextrin ist.

4. Zusammensetzung nach Anspruch 1, dadurch gekennzeichnet, daß der Zuckeralkohol Mannitol ist.

5. Verfahren zur Herstellung einer löslichen Zusammensetzung enthaltend Superoxid-Dismutase und Cyclodextrin, dadurch gekennzeichnet, daß zusätzlich mindestens ein Mono- oder Disaccharid oder ein von einem Monosaccharid abgeleiteter Zuckeralkohol verwendet wird.

6. Verfahren nach Anspruch 5, dadurch gekennzeichnet, daß der Zuckeralkohol Mannitol ist.

7. Verfahren nach Anspruch 5, dadurch gekennzeichnet, daß eine Lösung, enthaltend eine Superoxid-Dismutase, ein Cyclodextrin sowie zusätzlich mindestens ein Mono- oder Disaccharid oder einen von einem Monosaccharid abgeleiteten Zuckeralkohol, lyophilisiert wird.

8. Verfahren nach Anspruch 7, dadurch gekennzeichnet, daß der Gehalt der Lösung an Superoxid-Dismutase 0.1-60 mg/ml beträgt.

9. Verfahren nach Ansprüchen 7 oder 8, dadurch gekennzeichnet, daß die Superoxid-Dismutase eine Mangan-Superoxid-Dismutase ist.

10. Verfahren nach Anspruch 7, dadurch gekennzeichnet, daß das Cyclodextrin Hydroxypropyl-β-Cyclodextrin ist.

11. Verfahren nach Anspruch 10, dadurch gekennzeichnet, daß der Gehalt der Lösung an Hydroxypropyl-β-cyclodextrin 0.5-20% (Gewicht/Volumen) beträgt.

12. Verfahren nach Anspruch 10, dadurch gekennzeichnet, daß der Gehalt der Lösung an Hydroxypropyl-β-cyclodextrin 1-11% (Gewicht/Volumen) beträgt.

13. Verfahren nach Anspruch 10, dadurch gekennzeichnet, daß der Gehalt der Lösung an Hydroxypropyl-β-cyclodextrin 1-6% (Gewicht/Volumen) beträgt.

14. Verfahren nach Anspruch 7, dadurch gekennzeichnet, daß der Zuckeralkohol Mannitol ist.

15. Verfahren nach Anspruch 14, dadurch gekennzeichnet, daß der Gehalt der Lösung an Mannitol 0.5-10% (Gewicht/Volumen) beträgt.

16. Verfahren nach Anspruch 14, dadurch gekennzeichnet, daß der Gehalt der Lösung an Mannitol 1-5% (Gewicht/Volumen) beträgt.

17. Pharmazeutische Zusammensetzung, enthaltend Superoxid-Dismutase, Cyclodextrin, eine Substanz, die ausgewählt ist aus der Gruppe der Zucker oder Zuckeralkohole sowie pharmazeutisch geeignete Träger- und/oder Hilfsstoffe.

18. Pharmazeutische Zusammensetzung nach Anspruch 17, dadurch gekennzeichnet, daß die Superoxid-Dismutase eine Mangan-Superoxid-Dismutase ist.

19. Pharmazeutische Zusammnesetzung nach Anspruch 17, dadurch gekennzeichnet, daß das Cyclodextrin Hydroxypropyl-β-cyclodextrin ist.

20. Pharmazeutische Zusammensetzung, dadurch gekennzeichnet, daß der Zuckeralkohol Mannitol ist.

21. Pharmazeutische Zusammensetzung nach einem der Ansprüche 17 bis 20 zur Behandlung von Entzündungen, Lungenfibrose, bronchialer pulmonärer Dysplasie, Hyperoxie der Lunge, akuten Atemwegserkrankungen wie *adult respiratory distress syndrome* (ARDS) oder *infantile respiratory distress syndrome* (IRDS), Fibrose nach Strahlenexposition, durch Reperfusion nach Ischämie entstandenen Schäden, oder zur Verlängerung der Überlebenszeit isolierter Organ*e ex vivo.*

22. Verwendung der Zusammensetzung nach Ansprüchen 1 bis 4 zur Herstellung eines Arzneimittels für die Behandlung von Erkrankungen oder zur Prophylaxe bei Säugetieren und beim Menschen.

23. Verwendung von Superoxid-Dismutase, Hydroxypropyl-β-cyclodextrin und Mannitol zur Herstellung eines Arzneimittels für die Behandlung von Erkrankunegn oder zur Prophylaxe bei Säugetieren und beim Menschen.

24. Verwendung nach Anspruch 22 oder 23, dadurch gekennzeichnet, daß das Arzneimittel für die Behandlung von Entzündungen, Lungenfibrose, bronchialer pulmonärer Dysplasie, Hyperoxie der Lunge, akuten Atemwegserkrankungen wie *adult respiratory distress syndrome* (ARDS) oder *infantile respiratory distress syndrome* (IRDS), Fibrose nach Strahlenexposition, durch Repertusion nach Ischämie entstandenen Schäden, oder zur Verlängerung der Überlebenszeit isolierter Organe *ex vivo* ist.
